# FASCICULE DE BREVET EUROPEEN

(11) **EP 2 976 121 B1**
(45) Date de publication et mention de la délivrance du brevet: **26.07.2017**
(21) Numéro de dépôt: 14720644.5
(22) Date de dépôt: 18.03.2014
(51) Int. Cl.: A61M 15/00, A61M 15/08, B05B 11/02, A61M 5/24, B05B 11/00

(54) **DISPOSITIF DE DISTRIBUTION DE PRODUIT FLUIDE**
SPENDER FÜR EIN FLÜSSIGPRODUKT
FLUID PRODUCT DISPENSING DEVICE

(30) Priorité: 19.03.2013 FR 1352460
(43) Date de publication de la demande: 27.01.2016
(73) Titulaire: APTAR France SAS, 27110 Le Neubourg (FR)
(72) Inventeur: LE MANER, François, F-27400 La Vallée Montaure (FR); GREINER-PERTH, Jürgen, 78244 Gottmadingen (DE); CARRIÇO, Silvio, 78224 Singen (DE); WOCHELE, Matthias, 78239 Rielasingen-Worblingen (DE)
(74) Mandataire: CAPRI
(86) Numéro de dépôt international: PCT/FR2014/050618
(87) Numéro de publication internationale: WO 2014/147329

(56) Documents cités:
- EP-A1- 0 254 391
- EP-A1- 0 580 897
- FR-A1- 2 625 981
- US-A1- 2005 015 051
- US-B1- 6 708 846

## Description

La présente invention concerne un dispositif de distribution de produit fluide, et plus particulièrement un dispositif de type bidose.

Par dispositif de distribution de type bidose, on entend un dispositif contenant deux doses de produit fluide à distribuer lors de deux actionnements successifs du dispositif de distribution.

Les dispositifs de type bidose sont bien connus dans l'état de la technique. Ces dispositifs comportent généralement un réservoir contenant les deux doses de produit fluide à distribuer, et un organe de distribution, qui est généralement un piston, qui est monté coulissant dans ledit réservoir, et qui est déplacé pour distribuer le produit fluide contenu dans ledit réservoir. Lorsque le dispositif est un bidose, le déplacement du piston se fait en deux courses d'actionnement successives, de sorte qu'une première dose est distribuée lors d'un premier actionnement et une seconde dose est distribuée lors d'un deuxième actionnement. Avec ce type de dispositif de type bidose, il est parfois difficile pour l'utilisateur de savoir s'il a distribué une ou deux dose(s) avec son dispositif. Or, selon le type de produit fluide qui est distribué par le dispositif, notamment lorsqu'il s'agit d'un médicament, il peut être important d'éviter tout risque de sous-dosage et/ou de surdosage. Ainsi, par exemple, si le dispositif de type bidose est destiné à distribuer une dose respective dans chaque narine, il n'est généralement pas souhaitable que les deux doses soient distribuées dans la même narine. Or, un utilisateur qui aurait utilisé le dispositif pour distribuer une première dose dans sa première narine, et qui l'aurait ensuite reposé ou qui aurait été distrait, risquerait, s'il n'est pas certain d'avoir déjà utilisé le dispositif une première fois, de distribuer la seconde dose dans la même narine que la première dose. Ceci n'est généralement pas souhaitable. Ainsi, si l'on expulse deux fois le produit dans la même narine, l'excédent d'actif ne sera pas correctement absorbé par les tissus, voire repartira immédiatement de la narine en coulant, avec une perte évidente d'efficacité. De plus, aucune dose ne sera alors disponible pour la seconde narine. Les documents EP 0 254 391, FR 2 625 981, US 6 708 846, EP 0 580 897 et US 2005/015051 décrivent des dispositifs de l'état de la technique.

La présente invention a pour but de fournir un dispositif de distribution de produit fluide qui ne reproduit pas les inconvénients susmentionnés.

La présente invention a ainsi pour but de fournir un dispositif de distribution de produit fluide qui indique de manière fiable à l'utilisateur le nombre de doses distribuées.

La présente invention a également pour but de fournir un tel dispositif de distribution de produit fluide qui soit simple et peu coûteux à fabriquer et à assembler.

La présente invention a donc pour objet un dispositif de distribution de produit fluide comportant un réservoir contenant au moins deux doses de produit fluide, un organe de distribution, tel qu'un piston, monté coulissant dans ledit réservoir pour distribuer du produit fluide, une tête de distribution pourvue d'un orifice de distribution, ladite tête étant déplaçable par rapport audit réservoir pour déplacer ledit organe d'actionnement dans ledit réservoir et ainsi distribuer du produit fluide à travers ledit orifice de distribution, ladite tête de distribution comportant au moins deux fenêtres de visualisation, ledit dispositif comportant un indicateur mobile ensemble avec ledit réservoir, ledit indicateur coopérant après chaque actionnement du dispositif avec une fenêtre de visualisation respective.

Avantageusement, le réservoir contient deux doses de produit fluide, distribuées lors de deux actionnements successifs du dispositif, ladite tête de distribution comportant deux fenêtres de visualisation.

Avantageusement, ledit indicateur coopère avec une première fenêtre de visualisation après distribution de la première dose de produit fluide, et avec une seconde fenêtre de visualisation après distribution de la seconde dose de produit fluide.

Selon une première variante avantageuse, ledit indicateur comporte au moins une zone d'indication en couleur, ladite zone d'indication apparaissant dans ladite première fenêtre de visualisation après distribution de la première dose de produit fluide et dans la seconde fenêtre de visualisation après distribution de la seconde dose de produit fluide.

Selon une seconde variante avantageuse, ledit indicateur est adapté à masquer des zones d'indication en couleur prévues dans ladite tête de distribution, ledit indicateur masquant une zone d'indication en couleur dans ladite première fenêtre de visualisation après distribution de la première dose de produit fluide et masquant une zone d'indication en couleur dans ladite seconde fenêtre de visualisation après distribution de la seconde dose de produit fluide.

Avantageusement, après distribution de la seconde dose de produit fluide, ledit indicateur coopère avec les deux fenêtres de visualisation.

Avantageusement, ledit indicateur est formé sur un corps fixé audit réservoir.

Avantageusement, ledit indicateur est adapté à indiquer une distribution de dose incomplète à travers au moins une fenêtre de visualisation.

Ces avantages et caractéristiques et d'autres de la présente invention apparaîtront plus clairement au cours de la description détaillée suivante, faite en référence aux dessins joints, donnés à titre d'exemples non limitatifs, et sur lesquels

Les figures 1, 2 et 3 sont des vues schématiques en section transversale d'un dispositif de distribution de produit fluide selon une première variante de réalisation avantageuse de la présente invention, respectivement avant distribution de la première dose, après distribution de la première dose, et après distribution de la seconde dose,

La figure 4 est une vue agrandie du détail A de la figure 2,

La figure 5 est une vue agrandie du détail B de la figure 3,

Les figures 6 et 7 sont des vues schématiques partiellement en section transversale d'une seconde variante de réalisation de l'invention, respectivement après distribution de la première dose et après distribution de la seconde dose, et

Les figures 8 à 10 sont des vues schématiques de côté de la tête de distribution des figures 6 et 7, montrant l'indication visuelle visible par l'utilisateur, respectivement avant distribution de la première dose, après distribution de la première dose, et après distribution de la seconde dose.

La présente invention sera décrite ci-après en référence à un exemple de réalisation qui est un bidose, c'est-à-dire un dispositif contenant deux doses de produit fluide à distribuer lors de deux actionnements successifs du dispositif.

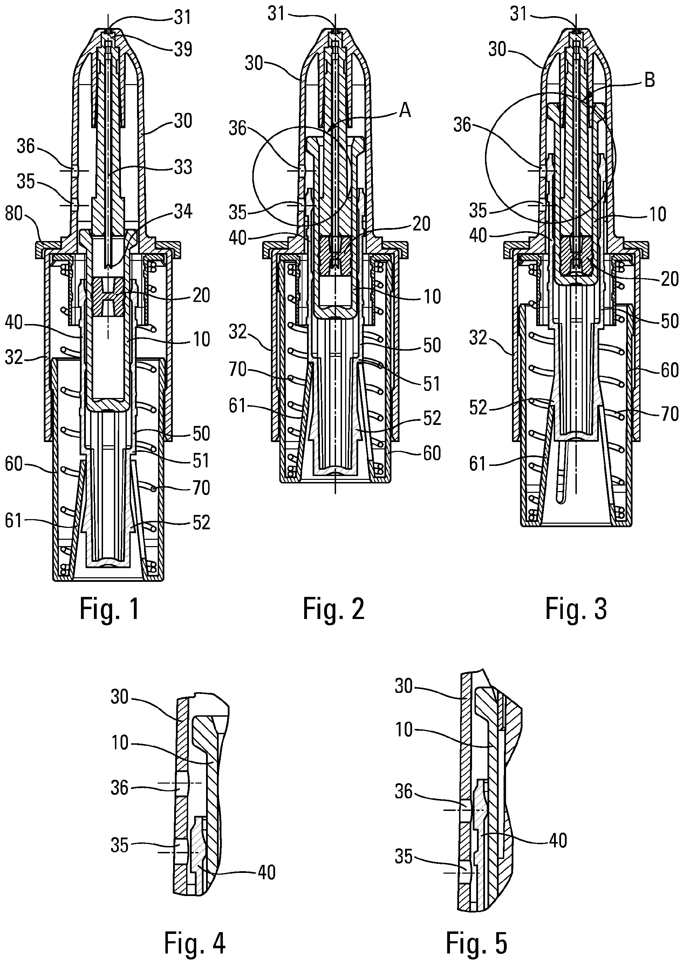

En se référant aux figures 1 à 3, le dispositif de distribution est un bidose qui comporte un réservoir 10 contenant deux doses de produit fluide. Un organe de distribution, tel qu'un piston 20, est monté coulissant dans ledit réservoir 10. Dans la position avant actionnement du dispositif, représentée sur la figure 1, ledit piston 20 fait office de bouchon en isolant le contenu du réservoir 10.

Une tête de distribution 30 est assemblée sur ledit réservoir 10 en étant déplaçable axialement par rapport à celui-ci. En particulier, un déplacement axial de la tête de distribution 30 par rapport au réservoir 10 provoque le déplacement du piston 20 dans le réservoir 10 et ainsi la distribution du produit fluide contenu dans ledit réservoir. La tête de distribution 30 comporte un canal de distribution 33 qui mène depuis une pointe de percement 34 jusqu'à l'orifice de distribution 31 de la tête de distribution 30. En amont de l'orifice de distribution 31, il peut être prévu un profil de pulvérisation 39, qui peut être d'un quelconque type connu et qui n'est pas représenté plus en détail sur les dessins, pour distribuer le produit fluide sous forme de spray.

Plus précisément, dans l'exemple représenté, le réservoir 10 est fixé dans un corps 50, qui est donc solidaire dudit réservoir 10, et qui se déplace ensemble avec lui.

La tête de distribution 30 comporte une jupe latérale inférieure 32 adaptée à coopérer avec un organe d'actionnement 60. Un élément repose-doigts 80 peut être assemblé autour de ladite tête de distribution 30, ou en variante être formé de manière monobloc avec elle.

Ledit organe d'actionnement 60 est axialement déplaçable à l'intérieur de ladite jupe latérale 32 de la tête de distribution 30 pour réaliser les actionnements successifs de dispositif. Comme visible notamment sur la figure 1, l'organe d'actionnement 60 comporte au moins une patte inclinée 61 qui est adaptée à coopérer avec des projections 51, 52 du corps 50 pour réaliser les actionnements successifs.

Un ressort de rappel 70 est monté entre l'organe d'actionnement 60 et la tête de distribution 30 pour ramener ledit organe d'actionnement 60 dans sa position de départ après chaque actionnement.

Le fonctionnement du dispositif représenté sur les figures 1 à 3 est le suivant. Dans la position de repos de la figure 1, le piston bouchon 20 isole le contenu du réservoir 10 de l'atmosphère. Lorsque l'utilisateur appuie simultanément sur le repose-doigt 80 et sur l'organe d'actionnement 60, il va déplacer ledit organe d'actionnement 60 à l'intérieur de la jupe latérale 32 de la tête de distribution 30. Ceci va pousser le corps 50 axialement vers le haut à partir de la position représentée sur la figure 1, par l'intermédiaire des pattes 61 qui vont pousser sur l'épaulement 51 dudit corps. Ceci va comprimer le ressort 70 et déplacer le réservoir 10 par rapport à la tête de distribution 30. Lorsque le réservoir 10 va commencer à se déplacer par rapport à la tête de distribution 30, l'extrémité de percement 34 du canal de distribution 33 va venir percer le piston de bouchon 20 pour faire communiquer l'intérieur du réservoir 10 avec ledit canal d'expulsion 33. Une poursuite de l'actionnement va provoquer un déplacement du piston 20 à l'intérieur du réservoir et donc une distribution de la première dose. Le produit fluide est donc poussé par ledit piston 20 à travers l'extrémité de percement 34 dans le canal de distribution 33, puis via le profil de pulvérisation 39, hors du dispositif à travers l'orifice de distribution 31.

Après distribution de la première dose, le dispositif est dans la position représentée sur la figure 2, et lorsque l'utilisateur relâche l'organe d'actionnement 60, le ressort 70 va ramener celui-ci vers sa position de départ. Pendant ce retour de l'organe d'actionnement 60, le réservoir et le corps 50 ne reviennent pas en arrière, car ces deux composants restent maintenus dans ladite tête de distribution 30. Eventuellement, la tête de distribution 30 peut comporter des moyens anti-retour pour empêcher un retour dudit corps 50 et/ou dudit réservoir 10 en arrière. Lorsque l'organe d'actionnement 60 revient sous l'effet du ressort de rappel 70 vers sa position de repos, les pattes 61 vont venir s'encliqueter sous la deuxième projection 52 du corps 50 ce qui va permettre à l'utilisateur d'actionner une deuxième fois le dispositif pour distribuer la deuxième dose de produit fluide.

La figure 3 représente la position après distribution de la deuxième dose.

Selon l'invention, le dispositif de distribution comporte un indicateur 40 pour indiquer à l'utilisateur la distribution de la première dose et la distribution de la deuxième dose. De cette manière, l'utilisateur sait exactement où il en est et si la première dose a ou non été distribuée. Cet indicateur 40 est adapté à coopérer avec des fenêtres de visualisation 35, 36 formées dans ladite tête de distribution 30. Avantageusement, ces fenêtres de visualisation 35, 36 sont formées dans une paroi latérale de la tête bien visible de l'utilisateur lorsqu'il tient le dispositif dans sa main. Bien entendu, si le nombre de doses de produit fluide contenues dans le réservoir est différent de deux alors le nombre de fenêtres de visualisation sera également différent de deux. Ces fenêtres de visualisation peuvent notamment être réalisées sous la forme de trous traversants à travers la paroi de la tête de distribution 30, comme illustré sur les figures.

Avantageusement, ledit indicateur 40 est formé sur ledit corps 50 qui est fixé au réservoir 10. Toutefois, en variante on pourrait imaginer un indicateur 40 formé directement sur le réservoir 10.

Selon une première variante avantageuse, cet indicateur 40 comporte au moins une zone colorée qui va venir s'afficher derrière les fenêtres de visualisation 35, 36 après les actionnements successifs du dispositif.

Ainsi, comme visible sur la figure 1, avant actionnement, aucune partie de l'indicateur 40 n'est visible derrière les fenêtres de visualisation 35, 36. Après distribution de la première dose, comme représenté sur la figure 2, l'indicateur 40 s'est déplacé par rapport à la tête de distribution 30 pour venir se placer derrière la première fenêtre de visualisation 35. Avantageusement, la zone colorée, typiquement en rouge, de l'indicateur 40 va donc indiquer à l'utilisateur par un affichage rouge dans la première fenêtre de visualisation 35 que la première dose a été distribuée, alors que l'affichage dans la seconde fenêtre de visualisation 36 reste inchangé. Après distribution de la seconde dose, ledit indicateur 40 a poursuivi son déplacement axial par rapport à la tête de distribution 30 pour venir se positionner derrière la seconde fenêtre de visualisation 36. Dans cette mise en oeuvre, après distribution de la deuxième dose, l'indicateur coopère avantageusement avec les deux fenêtres de visualisation 35, 36, c'est-à-dire que l'utilisateur verra la zone colorée dans les deux fenêtres 35, 36.

Ainsi pour résumer, avant le premier actionnement du dispositif, les deux fenêtres de visualisation 35, 36 ont un affichage qui apparaît vierge, par exemple en blanc. Après distribution de la première dose, la première fenêtre de visualisation 35 devient rouge, alors que la seconde fenêtre de visualisation 36 est toujours vierge. Après la distribution de la deuxième dose, les deux fenêtres de visualisation 35, 36 sont en rouge. L'utilisateur n'a donc aucune difficulté à voir très rapidement s'il a ou non distribué la première et/ou la seconde dose, et ne risquera donc pas de sur-doser et/ou sous-doser le produit, par exemple en distribuant le produit deux fois dans la même narine.

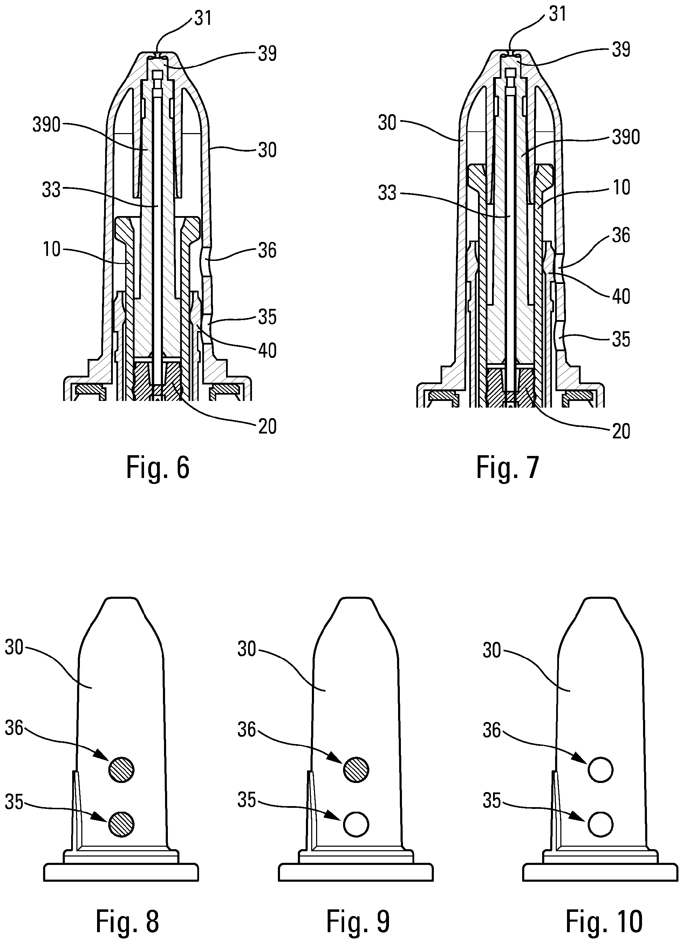

Selon une seconde variante avantageuse, l'indicateur 40 ne comporte pas de zone coloré, mais est au contraire réalisé avec la même couleur que la tête de distribution 30. Dans cette seconde variante, l'indicateur 40 est adapté à masquer des zones d'indication en couleur visibles à travers les fenêtres de visualisation 35, 36 avant actionnement. Ainsi, les zones d'indication en couleur peuvent être prévues sur une pièce solidaire de la tête de distribution, notamment sur l'élément de tige 390 qui est en contact avec le piston 20. En particulier, cet élément de tige 390 peut être entièrement réalisé en matériau coloré. Le réservoir 10 est alors avantageusement transparent pour permettre la visualisation desdites zones d'indication en couleur dans les fenêtres de visualisation 35, 36 avant actionnement. Avant le premier actionnement, les deux fenêtres de visualisation 35 et 36 affichent un point coloré, comme visible sur la figure 8. Après le premier actionnement, l'indicateur 40 masque la première fenêtre de visualisation 35, de sorte que seule la seconde fenêtre de visualisation 36 affiche un point coloré, comme visible sur la figure 9. Après le second actionnement, l'indicateur 40 masque les deux fenêtres de visualisation 35 et 36, de sorte qu'aucune fenêtre de visualisation n'affiche plus de point coloré, comme représenté sur la figure 10.

Ainsi pour résumer, avant le premier actionnement du dispositif, les deux fenêtres de visualisation 35, 36 ont un affichage coloré, par exemple en rouge ou en bleu. Après distribution de la première dose, la zone colorée derrière la première fenêtre de visualisation 35 est masqué, alors que l'affichage de la seconde fenêtre de visualisation 36 reste inchangé. Après la distribution de la deuxième dose, les zones colorées derrière les deux fenêtres de visualisation 35, 36 sont masquées par l'indicateur 40. L'utilisateur n'a donc aucune difficulté à voir très rapidement s'il a ou non distribué la première et/ou la seconde dose, et ne risquera donc pas de sur-doser et/ou sous-doser le produit, par exemple en distribuant le produit deux fois dans la même narine.

Avantageusement, l'indicateur 40 permet aussi d'indiquer une distribution incomplète, notamment en dimensionnant de manière appropriée les fenêtres de visualisation 35, 36. Ainsi, seule des distributions de doses complètes permettent à l'indicateur de remplir complètement la surface desdites fenêtres. Une distribution incomplète sera donc détectable par l'utilisateur grâce à une indication seulement partielle dans la fenêtre de visualisation concernée.

Bien entendu, la présente invention a été décrite en référence à deux variantes de réalisation, qui ne sont pas limitatives, et toute modification utile peut être apportée à la présente invention sans sortir du cadre de celle-ci tel que défini par les revendications annexées.

## Revendications

1. Dispositif de distribution de produit fluide comportant un réservoir (10) contenant deux doses de produit fluide, un organe de distribution (20), formé par un piston, monté coulissant dans ledit réservoir (10) pour distribuer du produit fluide, ledit piston étant déplaçable par rapport audit réservoir entre une position de repos, avant distribution de la première dose, et une position finale, après distribution de la seconde dose, une tête de distribution (30) pourvue d'un orifice de distribution (31), ladite tête étant déplaçable par rapport audit réservoir (10) pour déplacer ledit organe d'actionnement (20) dans ledit réservoir (10) et ainsi distribuer du produit fluide à travers ledit orifice de distribution (31), **caractérisé en ce que** ladite tête de distribution (30) comporte deux fenêtres de visualisation (35, 36), ledit dispositif comportant un indicateur (40) mobile ensemble avec ledit réservoir (10), ledit indicateur (40) coopérant après chaque actionnement du dispositif avec une fenêtre de visualisation respective (35, 36).

2. Dispositif selon la revendication 1, dans lequel ledit indicateur (40) coopère avec une première fenêtre de visualisation (35) après distribution de la première dose de produit fluide, et avec une seconde fenêtre de visualisation (36) après distribution de la seconde dose de produit fluide.

3. Dispositif selon la revendication 2, dans lequel ledit indicateur (40) comporte au moins une zone d'indication en couleur, ladite zone d'indication apparaissant dans ladite première fenêtre de visualisation (35) après distribution de la première dose de produit fluide et dans la seconde fenêtre de visualisation (36) après distribution de la seconde dose de produit fluide.

4. Dispositif selon la revendication 2, dans lequel ledit indicateur (40) est adapté à masquer des zones d'indication en couleur prévues dans ladite tête de distribution (30), ledit indicateur (40) masquant une zone d'indication en couleur dans ladite première fenêtre de visualisation (35) après distribution de la première dose de produit fluide et masquant une zone d'indication en couleur dans ladite seconde fenêtre de visualisation (36) après distribution de la seconde dose de produit fluide.

5. Dispositif selon l'une quelconque des revendications 2 à 4, dans lequel, après distribution de la seconde dose de produit fluide, ledit indicateur (40) coopère avec les deux fenêtres de visualisation (35, 36).

6. Dispositif selon l'une quelconque des revendications précédentes, dans lequel ledit indicateur (40) est formé sur un corps (50) fixé audit réservoir.

7. Dispositif selon l'une quelconque des revendications précédentes, dans lequel ledit indicateur (40) est adapté à indiquer une distribution de dose incomplète à travers au moins une fenêtre de visualisation (35, 36).

## Patentansprüche

1. Ausgabevorrichtung für ein fluides Produkt, aufweisend einen Behälter (10), der zwei Dosen von fluidem Produkt enthält, eine Ausgabeeinrichtung (20), die durch einen Kolben gebildet ist, der in dem Behälter (10) gleitend montiert ist, um fluides Produkt auszugeben, wobei der Kolben in Bezug auf den Behälter zwischen einer Ruheposition vor der Ausgabe der ersten Dosis und einer Endposition nach der Ausgabe der zweiten Dosis verschiebbar ist, einen Ausgabekopf (30), der mit einer Ausgabeöffnung (31) versehen ist, wobei der Kopf in Bezug auf den Behälter (10) verschiebbar ist, um die Betätigungseinrichtung (20) in dem Behälter (10) zu verschieben und so fluides Produkt durch die Ausgabeöffnung (31) auszugeben, **dadurch gekennzeichnet, dass** der Ausgabekopf (30) zwei Sichtfenster (35, 36) aufweist, wobei die Vorrichtung einen Anzeiger (40) aufweist, der zusammen mit dem Behälter (10) beweglich ist, wobei der Anzeiger (40) nach jeder Betätigung der Vorrichtung mit einem jeweiligen Sichtfenster (35, 36) zusammenwirkt.

2. Vorrichtung nach Anspruch 1, wobei der Anzeiger (40) nach der Ausgabe der ersten Dosis von fluidem Produkt mit einem ersten Sichtfenster (35) zusammenwirkt und nach der Ausgabe der zweiten Dosis von fluidem Produkt mit einem zweiten Sichtfenster (36) zusammenwirkt.

3. Vorrichtung nach Anspruch 2, wobei der Anzeiger (40) mindestens eine farbige Anzeigezone aufweist, wobei die Anzeigezone nach der Ausgabe der ersten Dosis von fluidem Produkt in dem ersten Sichtfenster (35) erscheint und nach der Ausgabe der zweiten Dosis von fluidem Produkt in dem zweiten Sichtfenster (36) erscheint.

4. Vorrichtung nach Anspruch 2, wobei der Anzeiger (40) dafür geeignet ist, in dem Ausgabekopf (30) vorgesehene farbige Anzeigezonen zu verdecken, wobei der Anzeiger (40) nach der Ausgabe der ersten Dosis von fluidem Produkt eine farbige Anzeigezone in dem ersten Sichtfenster (35) verdeckt und nach der Ausgabe der zweiten Dosis von fluidem Produkt eine farbige Anzeigezone in dem zweiten Sichtfenster (36) verdeckt.

5. Vorrichtung nach einem der Ansprüche 2 bis 4, wobei nach der Ausgabe der zweiten Dosis von fluidem Produkt der Anzeiger (40) mit den beiden Sichtfenstern (35, 36) zusammenwirkt.

6. Vorrichtung nach einem der vorhergehenden Ansprüche, wobei der Anzeiger (40) auf einem Körper (50) gebildet ist, der auf dem Behälter befestigt ist.

7. Vorrichtung nach einem der vorhergehenden Ansprüche, wobei der Anzeiger (40) dafür geeignet ist, eine unvollständige Dosisausgabe über mindestens ein Sichtfenster (35, 36) anzuzeigen.

## Claims

1. A fluid dispenser device comprising: a reservoir (10) containing two doses of fluid; a dispenser member (20), formed by a piston, that is mounted to slide in said reservoir (10) so as to dispense the fluid, said piston being movable relative to said reservoir between a rest position, before the first dose has been dispensed, and a final position, after the second dose has been dispensed; and a dispenser head (30) that is provided with a dispenser orifice (31), said head being movable relative to said reservoir (10) so as to move said actuator member (20) in said reservoir (10) and thus dispense the fluid through said dispenser orifice (31); the fluid dispenser device being **characterized in that** said dispenser head (30) includes two viewing windows (35, 36), said device including an indicator (40) that is movable together with said reservoir (10), said indicator (40) co-operating with a respective viewing window (35, 36) after each actuation of the device.

2. A device according to claim 1, wherein said indicator (40) co-operates with a first viewing window (35) after the first dose of fluid has been dispensed, and with a second viewing window (36) after the second dose of fluid has been dispensed.

3. A device according to claim 2, wherein said indicator (40) comprises at least one colored indication zone, said indication zone appearing in said first viewing window (35) after the first dose of fluid has been dispensed, and in the second viewing window (36) after the second dose of fluid has been dispensed.

4. A device according to claim 2, wherein said indicator (40) is adapted to mask colored indication zones that are provided in said dispenser head (30), said indicator (40) masking a colored indication zone in said first viewing window (35) after the first dose of fluid has been dispensed, and masking a colored indication zone in said second viewing window (36) after the second dose of fluid has been dispensed.

5. A device according to any one of claims 2 to 4, wherein, after the second dose of fluid has been dispensed, said indicator (40) co-operates with both viewing windows (35, 36).

6. A device according to any preceding claim, wherein said indicator (40) is formed on a body (50) that is fastened to said reservoir.

7. A device according to any preceding claim, wherein said indicator (40) is adapted to indicate, through at least one viewing window (35, 36), that an incomplete dose has been dispensed.
